# EUROPEAN PATENT APPLICATION

(11) **EP 4 506 333 A1**
(43) Date of publication of application: **12.02.2025**
(21) Application number: 23214598.7
(22) Date of filing: 06.12.2023
(51) Int. Cl.: C07C 303/22, C07C 309/14, C07C 303/44

(54) **METHOD FOR PREPARING TAURINE, METHYLTAURATE AND METHYLTAURINE SIMULTANEOUSLY**

(30) Priority: 07.08.2023 CN 202310990015
(71) Applicant: Qiangjiang Yongan Pharmaceutical Co., Ltd., Qianjiang, Hubei 433100 (CN)
(72) Inventor: Chen, Yong, Qianjiang, Hubei, 433100 (CN); Fang, Xiquan, Qianjiang, Hubei, 433100 (CN); Li, Shaobo, Qianjiang, Hubei, 433100 (CN); Zhou, Wei, Qianjiang, Hubei, 433100 (CN); Liu, Feng, Qianjiang, Hubei, 433100 (CN); Li, Xiang, Qianjiang, Hubei, 433100 (CN); Zhou, Wei, Qianjiang, Hubei, 433100 (CN)
(74) Representative: Gulde & Partner

(57) **Abstract**

The invention relates to a method for preparing taurine, methyltaurate and methyltaurine by using ethylene oxide, including: preparing taurine by an ethylene oxide process, and obtaining a taurine mother liquor; mixing the taurine mother liquor with methylamine, adding an alkaline catalyst, and carrying out an amination reaction to obtain a reaction solution; evaporating ammonia and methylamine from the reaction solution to obtain a methyltaurate solution; preparing a methyltaurine solution by using the methyltaurate solution; and concentrating and crystallizing the methyltaurine solution to obtain methyltaurine. The method of the invention avoids the problem of recycling a mother liquor in the existing taurine production to reduce the generation possibility of impurities, and meanwhile, can achieve quantitative conversion to methyltaurate and methyltaurine by controlling a molar ratio of raw materials to the catalyst in the reaction.

## Description

### TECHNICAL FIELD

The present invention relates to a method for preparing taurine, methyltaurate and methyltaurine, and particularly relates to a method for preparing taurine, methyltaurate and methyltaurine simultaneously.

### BACKGROUND

Taurine, chemically known as 2-aminoethanesulfonic acid, is the most abundant sulfur-containing free amino acid in cells of the body. A process route for chemical synthesis of taurine mainly includes an ethylene oxide process and an ethanolamine process. Preparation by the ethylene oxide process includes the following three steps.

Ethylene oxide and sodium bisulfite are subjected to an addition reaction by taking ethylene oxide as a starting material to obtain sodium isethionate. Sodium isethionate is subjected to ammonolysis to obtain sodium taurate. Sodium taurate is acidified by using, for example, hydrochloric acid, sulfuric acid, ion exchange resin, or electrodialysis, to obtain taurine, and separation and purification are then performed to obtain a product. The main reaction formulas are:

*CH*₂*CH*₂*O* + *NaHSO*₃ → *HOCH*₂*CH*₂*SO*₃*Na*;

*HOCH*₂*CH*₂*SO*₃*Na* + *NH*₃ → *H*₂*NCH*₂*CH*₂*SO*₃*Na* + *H*₂*O*;

*H*₂*NCH*₂*CH*₂*SO*₃*Na* + *H⁺* → *H*₂*NCH*₂*CH*₂*SO*₃*H* + *Na⁺*;

aminolysis side reactions include:

2HOCH₂CH₂SO₃Na + NH₃ → HN(CH₂CH₂SO₃Na)₂ + 2H₂O;

3HOCH₂CH₂SO₃Na + NH₃ → N(CH₂CH₂SO₃Na)₃ + 3H₂O;

isethionic acid derivatives, taurine derivatives and the like are produced during ammonolysis. These substances produced in the prior art are recycled in an ammonolysis reaction with a mother liquor to increase the yield, but at the same time, this is accompanied by increased energy consumption and increased production costs.

Sodium methyltaurate is a widely used fine chemical product, is a daily chemical intermediate and a textile aid, is also an important raw material for the production of surfactants, and may be also be used to synthesize a variety of high value-added products, such as sodium cocoyl-N-methyltaurate, a hydroxyethyl acrylate/sodium acryloyldimethyltaurate copolymer, and sodium polyacryloyldimethyltaurate, which may also be used as mild surfactants due to their mild features. The demands for the mild surfactants are increased greatly as the living standards of people are increasingly improved.

The synthesis of sodium methyltaurate is the most critical reaction for the mild surfactants in the sodium methyltaurate series. Methods for synthesis of sodium methyltaurate, which have now been disclosed, are as follows: a direct methylation method, a method for reconversion to a methylated product through an oxazolidinone intermediate, and a reductive amination method. Among these methods, some methods will produce unnecessary by-products; some methods use raw materials with higher prices; some methods require relatively harsh conditions; and some methods have a large number of reaction steps, and are prone to unavoidable errors, thereby reducing the total reaction yield.

A patent CN110963946A provides a method for preparing sodium methyltaurate from sodium taurate, wherein sodium methyltaurate is prepared from sodium taurate and formaldehyde as raw materials by a two-step reaction under the action of a catalyst. However, this method includes a hydrogenation process, and is complicated in process, and involves a hazardous process: a hydrogenation reaction.

In a patent CN113801039A, sodium methyltaurate is prepared by using sodium taurate and halomethane as raw materials and taking a quaternary ammonium salt as a catalyst. However, in this method, halogen atoms are newly introduced in a reaction system to result in a risk of corrosion of equipment, and the introduction of the halogen atoms also generates more waste water, which is not easy to treat.

In a patent CN115403487A, sodium methyltaurate is prepared by using sodium taurate and methylamine as raw materials. This method requires preparation of sodium taurate, followed by a reaction of sodium taurate with methylamine at a high temperature and a high pressure. This method requires the preparation of sodium taurate at a high pressure, followed by the preparation of sodium methyltaurate at a high temperature and a high pressure, which greatly increases energy consumption and results in higher production costs.

In conclusion, there are still the problems of complicated process routes, higher comprehensive costs and low yields in the current industrial production for synthesis of taurine and sodium methyltaurate.

### SUMMARY

The present invention aims at providing a low-cost and high-yield method for preparing taurine, methyltaurate and methyltaurine simultaneously.

The present invention provides a method for preparing taurine, methyltaurate and methyltaurine by using ethylene oxide, including: preparing taurine by an ethylene oxide process, and obtaining a taurine mother liquor; mixing the taurine mother liquor with methylamine, adding an alkaline catalyst, and carrying out an amination reaction to obtain a reaction solution; evaporating ammonia and methylamine from the reaction solution to obtain a methyltaurate solution; preparing a methyltaurine solution by using the methyltaurate solution; and concentrating and crystallizing the methyltaurine solution to obtain methyltaurine.

According to an example of the method of the present invention, a methyltaurine mother liquor obtained after concentrating and crystallizing the methyltaurine solution is subjected to cyclic concentration and extraction to obtain methyltaurine.

According to an example of the method of the present invention, the step of preparing taurine by the ethylene oxide process, and obtaining the taurine mother liquor includes: carrying out an addition reaction between ethylene oxide and a bisulfite solution to obtain isethionate; mixing the obtained isethionate with ammonia, adding an alkaline catalyst, and carrying out a reaction so as to obtain an ammonolysis reaction solution; evaporating the ammonolysis reaction solution after an ammonolysis reaction to remove excess ammonia so as to obtain a taurate solution; preparing a taurine solution by using the taurate solution; and concentrating and crystallizing the taurine solution to separate taurine, and obtain the taurine mother liquor.

According to an example of the method of the present invention, ammonia and methylamine are evaporated from the reaction solution, and then ammonia and methylamine are separated respectively; ammonia is reused in an aminolysis step for preparing taurine from ethylene oxide, and methylamine separated is used as a raw material for the amination reaction by being mixed with the taurine mother liquor.

According to an example of the method of the present invention, taurine, isethionate, ditaurate, tritaurate, taurine derivatives and isethionic acid derivatives in the taurine mother liquor react with methylamine in the presence of the alkaline catalyst. A molar weight of the alkaline catalyst is at least 90% of a molar weight of methyltaurate that can be correspondingly produced from taurine, isethionate, ditaurate, tritaurate, the taurine derivatives and the isethionic acid derivatives in the taurine mother liquor, and a preferred ratio of the molar weights is 1: 1 to 1.5:1.

According to an example of the method of the present invention, the alkaline catalyst is alkali metal hydroxide, alkali metal carbonate or alkali metal sulfite, is preferably the alkali metal hydroxide, and is most preferably sodium hydroxide.

According to an example of the method of the present invention, after the taurine mother liquor is mixed with methylamine, and the alkaline catalyst is added, the amination reaction is carried out at a temperature of 120°C-280°C, preferably at a temperature of 160°C-260°C.

According to an example of the method of the present invention, the taurine mother liquor and methylamine are subjected to the amination reaction in the presence of the alkaline catalyst for 1 min-60 min, preferably for 10 min-30 min.

According to an example of the method of the present invention, a pH for a reaction for preparing the methyltaurine solution by using the methyltaurate solution is controlled to be 5-7, or an alkali metal content of methyltaurate is controlled.

According to an example of the method of the present invention, the step of evaporating ammonia and methylamine from the reaction solution includes: carrying out flash evaporation on the reaction solution to separate ammonia from the reaction solution, and carrying out evaporation for concentration to separate methylamine from the reaction solution; or evaporating ammonia and methylamine from the reaction solution together, and then carrying out rectification to separate ammonia and methylamine.

According to an example of the method of the present invention, the step of evaporating ammonia and methylamine from the reaction solution includes: carrying out flash evaporation on the reaction solution, controlling the reaction solution to have a temperature of 150°C-200°C and a pressure of 1.4 Mpa-2.1 Mpa to separate ammonia from the reaction solution, and then carrying out evaporation for concentration to separate methylamine from the reaction solution.

According to an example of the method of the present invention, taurine is prepared from the taurate solution by a neutralization method, an ion exchange method, an ion membrane method or heating.

According to an example of the method of the present invention, methyltaurine is prepared from the methyltaurate solution by a neutralization method, an ion exchange method, an ion membrane method or heating.

According to an example of the method of the present invention, methyltaurate is lithium methyltaurate, sodium methyltaurate, potassium methyltaurate, calcium methyltaurate, or magnesium methyltaurate.

According to an example of the method of the present invention, taurine, isethionate, ditaurate, tritaurate, taurine derivatives and isethionic acid derivatives in the taurine mother liquor react with methylamine in the presence of the alkaline catalyst. A molar weight of methylamine is greater than or equal to a molar weight of methyltaurate that can be correspondingly produced from taurine, isethionate, ditaurate, tritaurate, the taurine derivatives and the isethionic acid derivatives in the taurine mother liquor, and a preferred ratio of the molar weights is 1: 1 to 1.5:1.

According to the preparation method of the present invention, taurine, methyltaurate and methyltaurine are prepared simultaneously by using ethylene oxide. The process is simple, methyltaurate and methyltaurine are prepared by fully using the remaining mother liquor in the production of taurine as a raw material, thereby avoiding the problem of recycling the mother liquor in the existing taurine production, and reducing the generation possibility of impurities. Quantitative conversion to methyltaurate and methyltaurine may be achieved by controlling the molar ratio of raw materials to the catalyst in the reaction, and an overall yield is 98% or above, not only improving the product quality of taurine and methyltaurate, but also greatly reducing the overall production cost. Therefore, the method is a green process route.

### BRIEF DESCRIPTION OF THE DRAWINGS

FIG 1 is a flowchart of a method for preparing taurine, methyltaurate and methyltaurine; and
FIG 2 is another flowchart of a method for preparing taurine, methyltaurate and methyltaurine.

### DETAILED DESCRIPTION

To make the objectives, contents and advantages of the present invention clearer, specific examples of the present invention will be further described in detail below with reference to the accompanying drawings and examples.

Raw materials used in the following examples are all commercially available products unless otherwise specified. The methods used are conventional methods unless otherwise specified. The contents of the materials refer to a mass/volume percent unless otherwise specified. HPLC and LC-MS analyze, show and detect the contents of substances in a reaction. The examples are described by taking sodium salts as examples, and actually, other salts of alkali metals such as potassium and lithium may also be used instead of the sodium salts.

It is found that under certain reaction conditions, a mother liquor in the production of taurine can be converted into alkali metal methyltaurate, and can be almost quantitatively converted after the long-term analysis and experimental studies on components of a taurine mother liquor by the inventor. It is found that the following factors are present in the conversion of alkali metal methyltaurate through the studies on reaction conditions and yields of reaction products by the inventor:
a mother liquor after taurine extraction contains a certain amount of taurine and isethionate, which can react with methylamine to prepare methyltaurate.

It is more surprising to the inventor that taurine derivatives and isethionic acid derivatives in the taurine mother liquor may also be quantitatively converted into methyltaurate upon addition of methylamine in the presence of a sufficient amount of alkaline catalyst.

Upon analysis by the inventor, it is found that a main reason is that methylamine has the strongest alkalinity and meanwhile is less sterically hindered, thereby being most favorable for the occurrence of a reaction. Additionally, the alkaline catalyst may be an alkaline compound such as alkali metal hydroxide, preferably sodium hydroxide.

It is found by further studies of the inventor that a molar weight of an alkali metal added should be greater than a total molar weight of taurine, isethionate, taurine derivatives, and isethionic acid derivatives in the taurine mother liquor. In addition to alkali metal hydroxide, alkali metal carbonate, alkali metal sulfite, and the like can be selected.

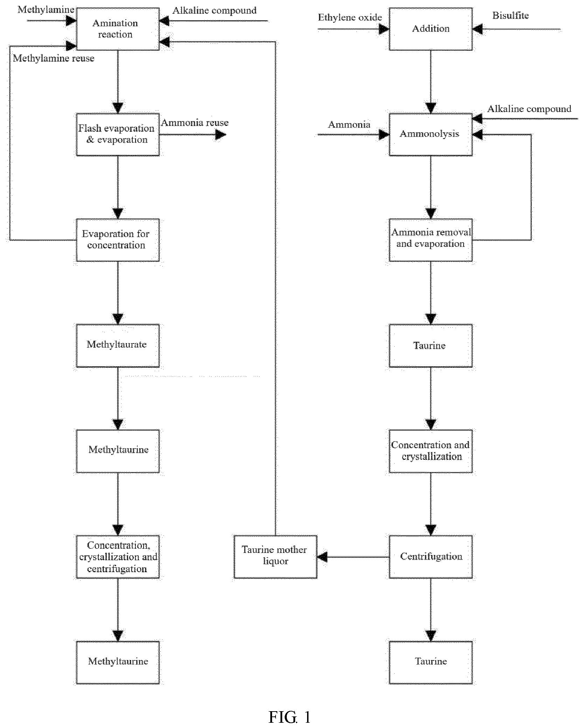

Based on the above experimental findings of the inventor, the present invention provides a method for preparing taurine, methyltaurate and methyltaurine by using ethylene oxide, as shown in FIG 1, specifically including the following steps:
Step S1: Ethylene oxide and a bisulfite solution are subjected to an addition reaction to obtain isethionate, wherein
   a reaction formula is as follows by taking sodium bisulfite as an example:

   *CH*₂*CH*₂*O* + *NaHSO*₃ → *HOCH*₂*CH*₂*SO*₃*Na.*
Step S2: The obtained isethionate in S1 is mixed with ammonia to be subjected to an ammonolysis reaction in the presence of alkaline to obtain an ammonolysis reaction solution, wherein
   a reaction formula is as follows by taking sodium isethionate as an example:

   *HOCH*₂*CH*₂*SO*₃*Na* + *NH*₃ → *H*₂*NCH*₂*CH*₂*SO*₃*Na* + *H*₂*O*.
Step S3: Excess ammonia is removed by evaporation after the ammonolysis reaction to obtain taurate.
Step S4: The obtained taurate is converted into taurine.

Specifically, a taurate solution can be converted into taurine by a neutralization method, an ion exchange method, an ion membrane method or heating.

A reaction formula is as follows by taking sodium taurate as an example:

*H*₂*NCH*₂*CH*₂*SO*₃*Na* + *H⁺* → *H*₂*NCH*₂*CH*₂*SO*₃*H* + *Na⁺*;

Step S5: A taurine solution is separated by concentration and crystallization to obtain taurine and a taurine mother liquor.

Step S6: The taurine mother liquor and methylamine are mixed to be subjected to a reaction in the presence of an alkaline catalyst to obtain a reaction solution.

Taking the condition that a salt for the reaction in the taurine mother liquor is a sodium salt as an example, a chemical reaction formula for converting substances in the taurine mother liquor and methylamine into sodium methyltaurate in the presence of the alkaline catalyst includes:
(1) carrying out a reaction between sodium isethionate and methylamine to produce sodium methyltaurate and water:

   *HOCH*₂*CH2SO*₃*Na* + *CH*₃*NH*₂ → *CH*₃*NHCH*₂*CH*₂*SO*₃*Na* + *H*₂*O*;
(2) carrying out a reaction between sodium ditaurate and methylamine to produce sodium methyltaurate and ammonia:

   HN(CH₂CH₂SO₃Na)₂ + 2CH₃NH₂ → 2CH₃HNCH₂CH₂SO₃Na + NH₃
(3) carrying out a reaction between sodium taurate and methylamine to produce sodium methyltaurate and ammonia:

   *NH*₂*CH*₂*CH*₂*SO*₃*Na* + *CH*₃*NH*₂ → *CH*₃*NHCH*₂*CH*₂*SO*₃*Na* + *NH*₃;
(4) carrying out a reaction between sodium tritaurate and methylamine to produce sodium methyltaurate and ammonia:

   N(CH₂CH₂SO₃Na)₃ + 3CH₃NH₂ → 3CH₃HNCH₂CH₂SO₃Na + NH₃;
(5) carrying out a reaction between taurine derivatives, sodium isethionate derivatives and methylamine to produce sodium methyltaurate and a corresponding product shown in the following general formula, wherein R represents different groups, *Rₘ*(*CH*₂*CH*₂*SO*₃*Na*)*ₙ* represents the taurine derivatives and the sodium isethionate derivatives:

   *Rₘ*(*CH*₂*CH*₂*SO*₃*Na*)*ₙ* + *nCH*₃*NH*₂ → *nCH*₃*NHCH*₂*CH*₂*SO*₃*Na* + *mRHₙ.*

Step S7: The reaction solution is subjected to flash evaporation after the reaction in the step S6 to remove and separate ammonia and methylamine so as to obtain an aqueous methyltaurate solution,
wherein ammonia separated may be further reused, and for example, may be reused in the step S2; and wherein methylamine separated may be reused in the step S6.

Step S8: Methyltaurate obtained is converted into methyltaurine.

Specifically, a methyltaurate solution may be converted into methyltaurine by a neutralization method, an ion exchange method, an ion membrane method or heating.

A specific reaction formula may be:

*CH*₃*NHCH*₂*CH*₂*SO*₃*Na* + *H⁺* → *CH*₃*H*₂*NCH*₂*CH*₂*SO*₃*H* + *Na*⁺.

Step S9: A methyltaurine solution is concentrated and crystallized to obtain methyltaurine.

A mother liquor obtained by separation may continue to be subjected to cyclic concentration and extraction in the step S9 to obtain methyltaurine.

For a preferred example, in the step S6, a molar weight of the alkaline catalyst is at least 90% of a molar weight of taurine, isethionate, ditaurate, tritaurate, the taurine derivatives and the isethionic acid derivatives (all being converted to a molar weight of sodium isethionate) in the taurine mother liquor, and a preferred ratio of the molar weights is 1: 1 to 1.5:1. Specifically, reference may be made to the chemical formulas in which the substances may produce methyltaurate with the corresponding molar weight, so that the converted molar weight of sodium isethionate may correspond to a molar weight of methyltaurate that can be produced.

For a preferred example, in the step S6, the alkaline catalyst is alkali metal hydroxide, alkali metal carbonate or alkali metal sulfite, is preferably the alkali metal hydroxide, and is most preferably sodium hydroxide.

For a preferred example, in the step S6, a molar ratio of methylamine to taurine, isethionate, ditaurate, tritaurate, the isethionate derivatives and the taurine derivatives (all being converted to a molar weight of sodium isethionate) in the taurine mother liquor is greater than or equal to 1:1, and is preferably 1:1 to 5:1.

For a preferred example, in the step S6, the amination reaction is carried out at a temperature of 120°C-280°C, preferably at a temperature of 160°C-260°C.

For a preferred example, in the step S6, the amination reaction is carried out for 1 min-60 min, preferably for 10 min-30 min.

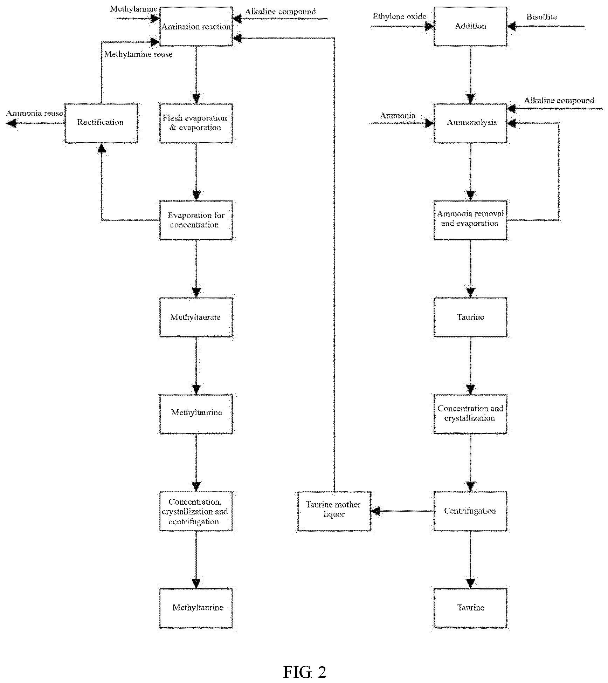

As shown in FIG 1 and FIG 2, in the step S7, after the amination is completed, flash evaporation is carried out first to separate ammonia for reuse, and then evaporation is carried out for concentration to remove methylamine and reuse methylamine in the amination reaction. Alternatively, ammonia and methylamine may be evaporated together, and then rectification is carried out to separate ammonia and methylamine. Subsequently, ammonia and methylamine are reused separately.

If flash evaporation is carried out after the amination is completed, ammonia is separated under the following reaction conditions: the reaction solution is controlled to have a temperature of 150°C-200°C, and a pressure of 1.4 Mpa-2.1 Mpa so as to avoid evaporation of methylamine.

In the step S8, a pH for conversion of methyltaurate to methyltaurine is controlled to be 5-7, or an alkali metal content of methyltaurate may also be controlled.

The technical effects of the present invention are demonstrated below by several different sets of experiments.

Example 1: This example shows an experiment for preparing sodium methyltaurate at different temperatures in the presence of sodium hydroxide.

2 mol of a sodium bisulfite solution and 2 mol of ethylene oxide were subjected to a reaction at a pressure of 0.1 Mpa, a pH of 5.0-9.0, and a temperature of 60°C-80°C to prepare sodium isethionate. Then, sodium isethionate, sodium hydroxide and ammonia were subjected to a reaction for 45 min at a temperature of 250°C-270°C and a pressure of 10 MPa-15 MPa. After the reaction was finished, a solution obtained after ammonia was removed was a sodium taurate solution. Sodium taurate was converted into taurine by using ion exchange resin, and then concentration and cooling crystallization were carried out to obtain 182 g of taurine. Taurine was 99.9% by mass. The remaining taurine mother liquor was 144 mL, wherein the content of taurine was 11.6% by mass/volume percent, and was 0.13 mol after being converted to a molar weight; the content of sodium isethionate was 14.8% by mass/volume percent, and was 0.14 mol after being converted to a molar weight; the content of ditaurine was 17.1% by mass/volume percent, and was 0.11 mol after being converted to a molar weight; and the content of tritaurine was 4.7% by mass/volume percent, and was 0.02 mol after being converted to a molar weight. Then 0.56 mol of sodium hydroxide was added into the mother liquor, then 2.8 mol of methylamine was introduced into the mother liquor, and the content of methylamine in the reaction solution was controlled to be 25%-40%. The reaction solution was heated to different temperatures in Table 1, and a reaction was carried out for 30 min. After the reaction was finished, methylamine and ammonia were removed from the solution by evaporation to obtain sodium methyltaurate.

**Table 1 Molar weights of substances after a reaction at different temperatures**

| Temperature (°C) | Sodium methyltaurate (mol) | Sodium taurate (mol) | Sodium isethionate (mol) | Sodium ditaurate (mol) | Sodium tritaurate (mol) | Overall yield (based on ethylene oxide) |
|---|---|---|---|---|---|---|
| 120 | 0.52 | 0 | 0 | 0 | 0.01 | 98.73% |
| 160 | 0.53 | 0 | 0 | 0 | 0.01 | 99.23% |
| 200 | 0.54 | 0 | 0 | 0 | 0 | 99.73% |
| 240 | 0.54 | 0 | 0 | 0 | 0 | 99.73% |
| 260 | 0.53 | 0 | 0.01 | 0 | 0 | 99.23% |
| 280 | 0.52 | 0.01 | 0.01 | 0 | 0 | 98.73% |

Example 2: This example shows an experiment for preparing sodium methyltaurate from a taurine mother liquor under the condition of different addition amounts of alkali metal catalysts.

A taurine mother liquor was prepared by the method in the above Example 1. Different catalysts were added into the mother liquor, then 2.8 mol of methylamine was introduced into the mother liquor, and the content of methylamine in a reaction solution was controlled to 25% to 40%. The reaction solution was heated to 200°C-240°C, and a reaction was carried out for 30 min. After completion of the reaction, methylamine and ammonia were removed from the solution by evaporation to obtain sodium methyltaurate.

**Table 2 Molar weight of sodium methyltaurate after a reaction in the presence of different alkali metals**

| Alkali metal | Alkali metal addition amount (mol) | Sodium methyltaurate (mol) |
|---|---|---|
| None | 0 | 0.05 |
| Sodium hydroxide | 0.1 | 0.15 |
| Sodium hydroxide | 0.25 | 0.2 |
| Sodium hydroxide | 0.5 | 0.54 |
| Sodium hydroxide | 0.6 | 0.55 |
| Sodium hydroxide | 0.75 | 0.55 |
| Sodium carbonate | 0.6 | 0.54 |
| Sodium sulfite | 0.6 | 0.5 |
| Potassium hydroxide | 0.6 | 0.55 |
| Potassium carbonate | 0.6 | 0.52 |
| Potassium sulfite | 0.6 | 0.5 |
| Lithium hydroxide | 0.6 | 0.53 |
| Lithium carbonate | 0.6 | 0.51 |

Example 3: This example shows an experiment for preparing sodium methyltaurate from a taurine mother liquor under the condition of different methylamine addition amounts.

A taurine mother liquor was prepared by the method in the above Example 1. 0.56 mol of sodium hydroxide was added into the mother liquor, then different amounts of methylamine were introduced into the mother liquor, and the content of methylamine in a reaction solution was controlled to be 25%-40%. The reaction solution was heated to 200°C-240°C, and a reaction was carried out for 30 min. After completion of the reaction, methylamine and ammonia were removed from the solution by evaporation to obtain sodium methyltaurate.

**Table 3: Molar weight of sodium methyltaurate after a reaction with different methylamine addition amounts**

| Methylamine addition amount (mol) | Sodium methyltaurate (mol) |
|---|---|
| 0.2 | 0.15 |
| 0.56 | 0.5 |
| 1 | 0.52 |
| 1.5 | 0.53 |
| 2 | 0.54 |
| 2.5 | 0.55 |
| 3 | 0.55 |
| 3.5 | 0.55 |
| 5 | 0.55 |
| 10 | 0.55 |
| 15 | 0.55 |

Example 4: This example shows an experiment for further preparing methyltaurine subsequently, which is as follows.

A taurine mother liquor was prepared by the method in the above example. 0.56 mol of sodium hydroxide was added into the mother liquor, then 2.8 mol of methylamine was introduced into the mother liquor, and the content of methylamine in a reaction solution was controlled to 25%-40%. The reaction solution was heated to 200°C-240°C, and a reaction was carried out for 30 min. After completion of the reaction, methylamine and ammonia were removed from the solution by evaporation to obtain sodium methyltaurate. Sodium methyltaurate was converted into methyltaurine in different manners (sulfuric acid neutralization, ion exchange, and an electrode membrane), a pH of a methyltaurine solution was 5-7, and then concentration and crystallization, centrifugation and drying were carried out to obtain methyltaurine. The contents of methyltaurine in the obtained samples were detected to be greater than 99.5%.

In conclusion, the method according to the present invention achieves the simultaneous preparation of taurine, methyltaurate and methyltaurine by sufficiently using the mother liquor produced from taurine production. Moreover, all the materials are recycled without discharge, achieving 100% utilization of the raw materials, and meanwhile, finally achieving an overall yield of 98% or above. In addition, the method also avoids the yield loss and energy waste caused by massively recycling the mother liquor in the original process, further saves consumption, and reduces comprehensive costs, thereby being an energy-saving and environment-friendly process route. The process provided by the present invention can be conducted discontinuously, semi-continuously, or continuously.

The above description is only the preferred examples of the present invention, and it should be noted that those of ordinary skill in the art may make multiple improvements and variations without departing from the technical principle of the present invention, and these improvements and variations should also fall within the scope of protection of the present invention.

## Claims

1. A method for preparing taurine, methyltaurate and methyltaurine by using ethylene oxide, **characterized by** comprising the following steps:
preparing taurine by an ethylene oxide process, and obtaining a taurine mother liquor;
mixing the taurine mother liquor with methylamine, adding an alkaline catalyst, and carrying out an amination reaction to obtain a reaction solution;
evaporating ammonia and methylamine from the reaction solution to obtain a methyltaurate solution;
preparing a methyltaurine solution by using the methyltaurate solution; and
concentrating and crystallizing the methyltaurine solution to obtain methyltaurine.

2. The method according to claim 1, **characterized in that** a methyltaurine mother liquor obtained after concentrating and crystallizing the methyltaurine solution is subjected to cyclic concentration and extraction to obtain methyltaurine.

3. The method according to claim 1, **characterized in that** the step of preparing taurine by the ethylene oxide process, and obtaining the taurine mother liquor comprises:
carrying out an addition reaction between ethylene oxide and a bisulfite solution to obtain isethionate;
mixing the obtained isethionate with ammonia, adding an alkaline catalyst, and carrying out a reaction so as to obtain an ammonolysis reaction solution;
evaporating the ammonolysis reaction solution after an ammonolysis reaction to remove excess ammonia so as to obtain a taurate solution;
preparing a taurine solution by using the taurate solution; and
concentrating and crystallizing the taurine solution to separate taurine, and obtain the taurine mother liquor.

4. The method according to claim 1, **characterized in that** ammonia and methylamine are evaporated from the reaction solution, and then ammonia and methylamine are separated respectively; ammonia is reused in an aminolysis step for preparing taurine from ethylene oxide, and methylamine separated is used as a raw material for the amination reaction by being mixed with the taurine mother liquor.

5. The method according to claim 1, **characterized in that** taurine, isethionate, ditaurate, tritaurate, taurine derivatives and isethionic acid derivatives in the taurine mother liquor react with methylamine in the presence of the alkaline catalyst; wherein
a molar weight of the alkaline catalyst is at least 90% of a molar weight of methyltaurate that can be correspondingly produced from taurine, isethionate, ditaurate, tritaurate, the taurine derivatives and the isethionic acid derivatives in the taurine mother liquor, and a preferred ratio of the molar weights is 1:1 to 1.5:1.

6. The method according to claim 1, **characterized in that** the alkaline catalyst is alkali metal hydroxide, alkali metal carbonate or alkali metal sulfite, is preferably the alkali metal hydroxide, and is most preferably sodium hydroxide.

7. The method according to claim 1, **characterized in that** after the taurine mother liquor is mixed with methylamine, and the alkaline catalyst is added, the amination reaction is carried out at a temperature of 120°C-280°C, preferably at a temperature of 160°C-260°C.

8. The method according to claim 1, **characterized in that** the taurine mother liquor and methylamine are subjected to the amination reaction in the presence of the alkaline catalyst for 1 min-60 min, preferably for 10 min-30 min.

9. The method according to claim 1, **characterized in that** a pH for a reaction for preparing the methyltaurine solution by using the methyltaurate solution is controlled to be 5-7, or an alkali metal content of methyltaurate is controlled.

10. The method according to claim 1, **characterized in that** the step of evaporating ammonia and methylamine from the reaction solution comprises:
carrying out flash evaporation on the reaction solution to separate ammonia from the reaction solution, and carrying out evaporation for concentration to separate methylamine from the reaction solution; or
evaporating ammonia and methylamine from the reaction solution together, and then carrying out rectification to separate ammonia and methylamine.

11. The method according to claim 1 or 10, **characterized in that** the step of evaporating ammonia and methylamine from the reaction solution comprises: carrying out flash evaporation on the reaction solution, controlling the reaction solution to have a temperature of 150°C-200°C and a pressure of 1.4 Mpa-2.1 Mpa to separate ammonia from the reaction solution, and then carrying out evaporation for concentration to separate methylamine from the reaction solution.

12. The method according to claim 3, **characterized in that** taurine is prepared from the taurate solution by a neutralization method, an ion exchange method, an ion membrane method or heating.

13. The method according to claim 1, **characterized in that** methyltaurine is prepared from the methyltaurate solution by a neutralization method, an ion exchange method, an ion membrane method or heating.

14. The method according to claim 1, **characterized in that** methyltaurate is lithium methyltaurate, sodium methyltaurate, potassium methyltaurate, calcium methyltaurate, or magnesium methyltaurate.

15. The method according to claim 1 or 5, **characterized in that** taurine, isethionate, ditaurate, tritaurate, taurine derivatives and isethionic acid derivatives in the taurine mother liquor react with methylamine in the presence of the alkaline catalyst; wherein
a molar weight of methylamine is greater than or equal to a molar weight of methyltaurate that can be correspondingly produced from taurine, isethionate, ditaurate, tritaurate, the taurine derivatives and the isethionic acid derivatives in the taurine mother liquor, and a preferred ratio of the molar weights is 1: 1 to 1.5:1.
